# EUROPÄISCHE PATENTSCHRIFT

(11) **EP 2 449 997 B1**
(45) Veröffentlichungstag und Bekanntmachung des Hinweises auf die Patenterteilung: **25.03.2020**
(21) Anmeldenummer: 11187650.4
(22) Anmeldetag: 03.11.2011
(51) Int. Cl.: A61B 34/30

(54) **Medizinischer Arbeitsplatz**
Medical workstation
Poste de travail médical

(30) Priorität: 08.11.2010 DE 102010043584
(43) Veröffentlichungstag der Anmeldung: 09.05.2012
(73) Patentinhaber: KUKA Deutschland GmbH, 86165 Augsburg (DE)
(72) Erfinder: Neff, Thomas, 80339 München (DE); Jacob, Dirk, 87616 Marktoberdorf (DE); Kuschel, Martin, 80339 München (DE); Ueberle, Marc-Walter, 86316 Friedberg (DE); Ortmaier, Tobias, 30966 Hemmingen (DE)
(74) Vertreter: Böss, Dieter Alexander

(56) Entgegenhaltungen:
- WO-A1-2009/151206
- US-A1- 2004 034 282
- US-A1- 2009 171 184
- US-B1- 6 325 808

## Beschreibung

Die Erfindung betrifft einen medizinischen Arbeitsplatz mit wenigstens einem Roboterarm.

In der Telepräsenz bzw. Teleaktion werden Roboter über relativ große Barrieren ferngesteuert. Dabei werden Sollkommandos von einer Bedienperson an einer Bedienvorrichtung bzw. Eingabekonsole sensoriell erfasst, verarbeitet und an den entfernt stehenden Roboter übertragen. Gegebenenfalls über einen visuellen Rückkanal kann die Bewegung des Roboters überwacht werden. Eine Anwendung solcher Systeme ist die telemanipulierte Chirurgie, bei der z.B. ein Arzt mittels seiner Eingabekonsole manuell mehrere Roboterarme bewegt, die z.B. ein medizinisches Instrument bewegen, um ein Lebewesen damit zu behandeln, insbesondere zu operieren. Die Eingabekonsole umfasst in der Regel eine 3D-Anzeigevorrichtung, welche aktuell aufgenommene 3D-Bilder vom Operationsgebiet zeigt. Mittels Eingabemittel z.B. in Form von Griffen kann der Arzt die Roboterarme telemanipulieren, d.h. bewegen, um damit das medizinische Instrument mittels der Roboterarme zu bewegen. Die WO 2010/025943 A1 offenbart ein Beispiel eines solchen medizinischen Arbeitsplatzes.

Die US 6,325,808 B1 offenbart einen medizinischen Arbeitsplatz mit zwei Docking Stationen, die mittels Ständer gestützt werden können. Die Docking Stationen sind zur Aufnahme eines medizinischen Instruments vorgesehen, welches mittels der Docking Station automatisch bewegt werden kann. Dazu ist ein Controller vorgesehen. Ein Chirurg kann eine Bahn für ein Nähen vorgeben, sodass der medizinische Arbeitsplatz in einem automatischen Betriebsmodus das Nähen automatisch vollzieht. Die Aufgabe der Erfindung ist es, einen verbesserten roboterunterstützten medizinischen Arbeitsplatz anzugeben.

Die Aufgabe der Erfindung wird gelöst durch einen medizinischen Arbeitsplatz zum Behandeln eines Lebewesens mittels eines medizinischen Instruments, aufweisend
- wenigstens ein Roboterarm, der mehrere mittels Gelenken verbundene Glieder, Antriebe zum Bewegen der Glieder, und eine Befestigungsvorrichtung aufweist, die vorgesehen ist, mit dem medizinischen Instrument zum Behandeln des Lebewesens versehen zu werden,
- wenigstens eine mit den Antrieben gekoppelte Steuervorrichtung, welche eingerichtet ist, Signale zum Ansteuern der Antriebe zu erzeugen, damit die Befestigungsvorrichtung den Signalen zugeordnete Bewegungen durchführt,
- eine mit der Steuervorrichtung gekoppelte Anzeigevorrichtung zum Anzeigen wenigstens eines während der Behandlung des Lebewesens aufgenommenen Bildes vom Behandlungsbereich des Lebewesens aufweist,
- eine Eingabeeinrichtung, mittels derer ein Bereich im Bild kennzeichenbar ist, wobei die Steuervorrichtung eingerichtet ist, in einem zweiten Betriebsmodus aufgrund des gekennzeichneten Bereichs automatisch eine Bewegung des relevanten Roboterarms zu errechnen und die Antriebe dieses Roboterarms entsprechend anzusteuern, sodass das medizinische Instrument mittels dieses Roboterarms automatisch den Bereich des Lebewesens behandelt, der dem gekennzeichneten Bereich zugeordnet ist, und
- eine mit der Steuervorrichtung gekoppelte Bedienkonsole, die eine mit der Steuervorrichtung gekoppelte manuelle Eingabevorrichtung aufweist, wobei die Steuervorrichtung in einem weiteren Betriebsmodus die Signale aufgrund eines manuellen Bewegens der Eingabevorrichtung erzeugt, sodass der Roboterarm eine der manuellen Bewegung entsprechende Bewegung durchführt, wobei
   bei dem medizinischen Arbeitsplatz wenigstens eine der manuellen Eingabevorrichtungen umschaltbar ausgeführt ist, sodass diese Eingabevorrichtung im weiteren Betriebsmodus zum telemanipulierten Bewegen und im Betriebsmodus zum Kennzeichnen des Bereichs verwendbar ist.

Der erfindungsgemäße medizinische Arbeitsplatz ist demnach derart eingerichtet, dass in dem mittels der Anzeigevorrichtung dargestellten Bildes ein Bereich gekennzeichnet werden kann. Das Bild stammt von dem Bereich des Lebewesens, das aktuell behandelt wird und ist vorzugsweise ein dreidimensionales Bild. Die Steuervorrichtung, die die Antriebe der Roboterarme ansteuert, ist eingerichtet, aufgrund des gekennzeichneten Bereichs eine Bewegung für den Roboterarm zu errechnen, an dem das medizinische Instrument befestigt ist. Diese Bewegung erfolgt ohne manuelles Bewegen des Roboterarms. Diese Bewegung ist derart, dass mittels des Roboterarms das medizinische Instrument derart im Behandlungsbereich bewegt wird, dass es automatisch das Lebewesen behandelt. Ist das medizinische Instrument z.B. zum Nähen vorgesehen, dann steuert die Steuervorrichtung den Roboterarm bzw. den relevanten Roboterarm und das medizinische Instrument derart an, dass das medizinische Instrument automatisch das Nähen vollzieht. In diesem Zusammenhang soll noch erwähnt sein, dass der Begriff "Steuern" weit gefasst sein soll und neben dem Steuern im engeren Sinn auch ein Regeln umfassen soll.

Die Anzeigevorrichtung kann auch Teil der Bedienkonsole sein. Gemäß dieser Variante ist der oder die Roboterarme mittels der Eingabevorrichtung der Bedienkonsole im weiteren Betriebsmodus telemanipulierbar, um somit das Lebewesen mittels des medizinischen Instruments zu behandeln.

Nach einer Variante des erfindungsgemäßen medizinischen Arbeitplatzes weist dieser eine Kamera auf, die vorgesehen ist, das Bild vom Behandlungsbereich des Lebewesens während der Behandlung zu erstellen. Die Kamera, die vorzugsweise als 3D-Kamera ausgebildet ist, kann insbesondere an der Befestigungsvorrichtung eines weiteren Roboterarms befestigt sein, um insbesondere telemanipuliert an den Behandlungsbereich bewegt zu werden.

Die Kamera kann vorzugsweise an einem Endoskop befestigt oder in ein solches integriert sein. Dann ist es möglich, die Kamera, bewegt durch den weiteren Roboterarm, in das Lebewesen einzuführen. Die mittels der Kamera aufgenommenen Bilder können dann auch dazu verwendet werden, gegebenenfalls die telemanipulierte Bewegung des medizinischen Instruments zu überwachen.

Um den Bereich besser zu kennzeichnen, kann das Bild ein dreidimensionales Bild sein. Die Anzeigevorrichtung kann vorzugsweise derart eingerichtet sein, dass sie ein dreidimensionales Bild darstellen kann.

Als Eingabeeinrichtung kann beispielsweise eine Rechner-Maus, insbesondere eine 3D-Maus verwendet werden. Auch komplexere Eingabeeinrichtungen, z.B. ein sogenannter Pen mit einem Schalter einem haptischen Eingabegerät mit 6 Freiheitsgraden gegebenenfalls mit Kraft-, Temperatur- und/oder Vibrationsrückkopplung, können als Eingabeeinrichtung verwendet werden.

Bei dem erfindungsgemäßen medizinischen Arbeitsplatz ist wenigstens eine der manuellen Eingabevorrichtungen umschaltbar ausgeführt, sodass diese Eingabevorrichtung während des weiteren Betriebsmodus zum telemanpulierten Bewegen und während des Betriebsmodus zum Kennzeichnen des Bereichs verwendbar ist.

Der erfindungsgemäße medizinische Arbeitsplatz kann eingerichtet sein, dass mit ihm verschiedene medizinische Instrumente telemanipulierbar sind. Mögliche medizinische Instrumente können beispielsweise zum Kleben, Nähen, Klammern, Schneiden oder Cautern, oder für eine Biopsie oder für die Brachytherapie vorgesehen sein.

Gemäß einer Ausführungsform des medizinischen Arbeitsplatzes kann dieser Eingabemittel aufweisen, die vorgesehen sind, die Art des medizinischen Instruments auszuwählen. Somit wird es ermöglicht, dass entsprechend der ausgewählten Art eine entsprechende Behandlung des gekennzeichneten Bereichs automatisiert durchgeführt wird.

Die Eingabemittel können beispielsweise auf Spracheingabe und/oder auf Gestenerkennung basieren.

Damit die Steuervorrichtung des erfindungsgemäßen medizinischen Arbeitsplatzes während des Betriebsmodus die automatische Bewegung besser planen kann, kann die Steuervorrichtung eingerichtet sein, die Bewegung des Roboterarms aufgrund von vor der Behandlung aufgenommener Bilder des Lebewesens und/oder aufgrund von anatomischen Atlanten zu errechnen. Die vor der Behandlung aufgenommenen Bilder können beispielsweise mittels eines bildgebenden medizintechnischen Gerätes aufgenommen werden, wie z.B. eines Magnetresonanzgerätes, eines Röntgengerätes oder eines Computertomographen.

Je nach Ausführungsform des erfindungsgemäßen medizinischen Arbeitsplatzes kann dieser beispielsweise einen Chirurgen von repetitiven, insbesondere ermüdenden Arbeiten entlasten und somit gegebenenfalls die Dauer der Behandlung des Lebewesens verkürzen. Dazu können autonome Funktionen zum Einsatz kommen, die "im Bild" z.B. vom Chirurgen definiert werden. Dies kann einerseits zu einer Kostenreduktion führen und andererseits eine geringere Belastung des Lebewesens, z.B. durch Narkose, zur Folge haben. Hierzu können die im Bild definierten Aufgaben durch den/ die Roboter automatisiert durchgeführt werden. Präoperative und intraoperative Daten können herangezogen werden.

So kann es z.B. vorgesehen sein, dass zunächst eine Aufgabe und/ oder die Lagen im Bild, das ein 2D, vorzugsweise ein 3D Bild ist, definiert wird, z.B. mittels einer 3D Maus, Gestenerkennung des Eingabegeräts, einer Kombination mit Spracheingabe oder mittels eines komplexeren Eingabegerätes (z.B. Pen mit einem Schalter an haptischen Eingabegerät mit 6 Freiheitsgraden mit Kraft-, Temperatur- und Vibrationsfeedback), die z.B. eine intuitivere Auswahl von Regionen bzw. Bereichen im Bild erlauben. Letzter erlaubt z.B. eine Realisierung eines virtuellen 6D Grafiktabletts. Es kann beispielsweise das bereits vorhandene Eingabegerät (Eingabevorrichtung der Bedienkonsole) verwendet werden (Umschalten zwischen den Funktionen "Bedienung des Teleoperators" und "Eingabegerät zur Aufgabendefinition").

Anschließend ergibt sich gegebenenfalls ein automatisches Anfahren von Punkten/Trajektorien bzw. Abfahren von Arealen durch den Roboter bzw. den Roboterarm.

Gegebenfalls kann eine Bewegungskompensation bzw. Aktualisierung der Szene durch ein 3D Endoskop, andere intraoperative Sensorik oder eine Kombination von Sensoren erfolgen.

Es können auch (segmentierte) präoperativen Daten oder Daten aus anatomischen Atlanten berücksichtigt werden.

Es können auch folgende Komponenten in den erfindungsgemäßen medizinischen Arbeitsplatz integriert werden:
- Lernen durch Vormachen,
- Kollisionsvermeidung bei kooperierenden Robotern,
- Bahnplanung zur Optimierung (zeit-/ kraftoptimiert).

Ein Ausführungsbeispiel der Erfindung ist exemplarisch in den beigefügten schematischen Figuren dargestellt. Es zeigen:
- Fig. 1: einen medizinischen Arbeitsplatz,
- Fig. 2: eine mittels eines Endoskops geführte Kamera und
- Fig. 3: eine Anzeigevorrichtung des medizinischen Arbeitsplatzes.

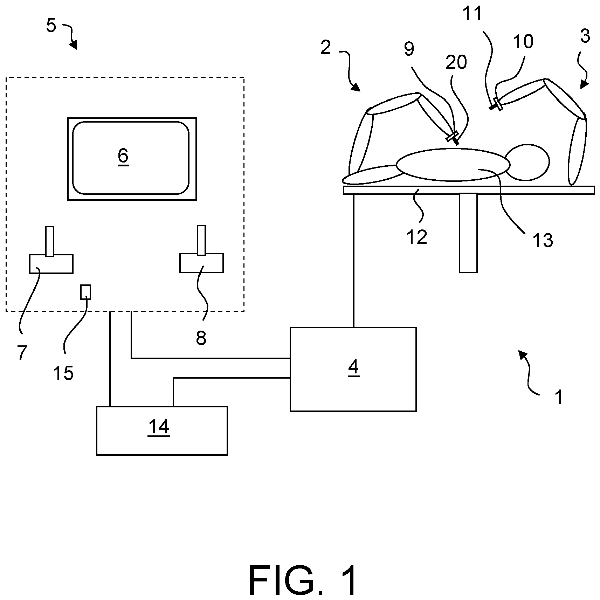

Die Fig. 1 zeigt einen medizinischen Arbeitsplatz 1, der mehrere Roboterarme 2, 3, eine Steuervorrichtung 4 und eine mit der Steuervorrichtung 4 gekoppelte Bedienkonsole 5 aufweist. Die Bedienkonsole 5 umfasst ein Anzeigevorrichtung 6, die insbesondere eingerichtet ist, dreidimensionale Bilder anzuzeigen, und manuelle Eingabevorrichtungen 7, 8, mittels derer eine nicht näher dargestellte Person, z.B. ein Chirurg, die Roboterarme 2, 3 in einem ersten Betriebsmodus telemanipulieren kann, wie dies im Prinzip dem Fachmann bekannt ist.

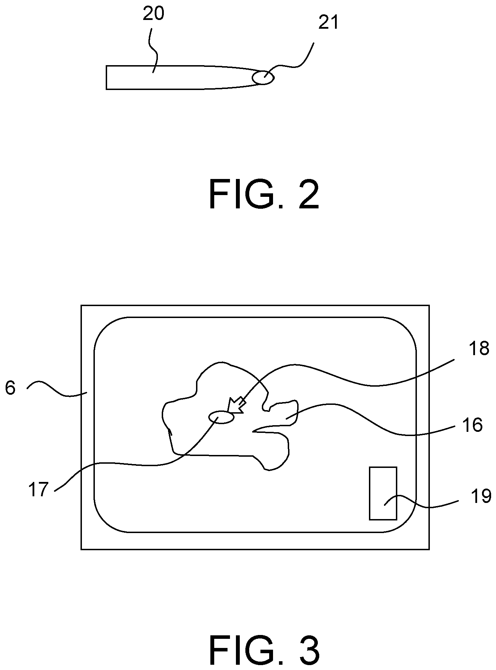

Im Falle des vorliegenden Ausführungsbeispiels umfasst jeder der Roboterarme 2, 3 mehrere Glieder, die über Gelenke verbunden sind, und eine Befestigungsvorrichtung 9, 10, an denen z.B. ein medizinisches Instrument 11 oder ein in der Fig. 2 näher dargestelltes Endoskop 20, in das insbesondere eine Kamera 21 integriert ist, befestigt werden kann. Die Antriebe der Roboterarme 2, 3, die insbesondere elektrische Antriebe sind, sind mit der Steuervorrichtung 4 verbunden. Die Steuervorrichtung 4, die z.B. als ein Rechner ausgeführt ist, ist eingerichtet, insbesondere mittels eines Rechenprogramms die Antriebe derart anzusteuern, sodass die Roboterarme 2, 3 ihre Befestigungsvorrichtungen 9, 10 und somit das medizinische Instrument 11 bzw. das Endoskop 20 gemäß einer mittels der manuellen Eingabevorrichtungen 7, 8 gewünschten Bewegung ausführen. Gegebenfalls ist die Steuervorrichtung 4 auch derart eingerichtet, dass sie die Bewegungen der Roboterarme 2, 3 bzw. der Antriebe regelt, sodass im vorliegenden Fall der Begriff "Steuern" auch "Regeln" umfassen soll.

Im Falle des vorliegenden Ausführungsbeispiels ist der medizinische Arbeitsplatz 1 vorgesehen, dass mit ihm ein z.B. auf einer Patientenliege 12 liegendes Lebewesen 13 insbesondere minimal invasiv mittels des medizinischen Instruments 11 behandelt werden kann. Das medizinische Instrument 11 ist z.B. ein Skalpell oder eine Vorrichtung zum Zunähen von Gewebe des Lebewesens 13 und kann im ersten Betriebsmodus telemanipuliert mittels des Roboterarms 3 in das Lebewesen 13 hineingeführt werden. Der medizinische Arbeitsplatz 1 kann auch mehr als zwei Roboterarme 2, 3 umfassen, wobei der oder die weiteren Roboterarme ebenfalls mit der Steuervorrichtung 4 verbunden sind und mittels der Bedienkonsole 5 telemanipulierbar sind. Am weiteren Roboterarm kann ebenfalls ein medizinisches Instrument befestigt sein.

Das medizinische Instrument 11 kann z.B. zum Kleben, Nähen, Klammern, Schneiden oder Cautern vorgesehen sein, oder für eine Biopsie oder für die Brachytherapie vorgesehen sein.

Im Falle des vorliegenden Ausführungsbeispiels kann der medizinische Arbeitsplatz 1 eine insbesondere mit der Steuervorrichtung 4 gekoppelte Datenbank 14 umfassen, in der z.B. präoperative Daten vom Lebewesen 13 und/oder anatomische Atlanten gespeichert sind. Die präoperativen Daten umfassen z.B. dem Lebewesen 13 zugeordnete präoperativ aufgenommene Bilddatensätze, die z.B. mittels eines medizintechnischen Gerätes vor der Behandlung aufgenommen wurden. Medizintechnische Geräte sind beispielsweise Computertomographie-Geräte, Magnetresonanzgeräte, etc. Die Datenbank 14 kann auch mit der Bedienkonsole 5 gekoppelt sein, sodass z.B. die präoperativen Daten und/oder die anatomischen Atlanten an der Anzeigevorrichtung 6 angezeigt werden können.

Um während der Behandlung des Lebewesens 13 Bilder 16 vom Operationssitus, also von dem Bereich des Lebewesens 13, der mittels des medizinischen Arbeitsplatzes 1 behandelt wird, an der Anzeigevorrichtung 6 der Bedienkonsole 5 darzustellen, ist im Falle des vorliegenden Ausführungsbeispiels die Kamera 21 vorgesehen. Diese ist z.B. mit der Steuervorrichtung 4 verbunden, sodass mittels der Kamera 21 aufgenommene Bilddatensätze, die insbesondere 3D-Bilddatensätze sind, als die Bilder 16 an der Anzeigevorrichtung 6 darstellbar sind. Ein Beispiel eines Bildes 16 ist in der Fig. 3 gezeigt.

Die Kamera 21 ist z.B. an einem Ende des Endoskops 20 befestigt oder an diesem Ende ins Endoskop 20 integriert. Mit dem anderen Ende ist das Endoskop 20 an der Befestigungsvorrichtung 9 des Roboterarms 2 befestigt, sodass mittels der Bedienkonsole 5 das Endoskop 20 und somit die Kamera 21 bewegt werden kann. Somit kann die das Lebewesen 13 behandelnde Person während der Behandlung geeignete Bilder 16 vom Operationssitus erstellen, die an der Anzeigevorrichtung 6 dargestellt werden. Die Bilder 16 vom Operationssitus sind insbesondere dreidimensionale Bilder.

Im Falle des vorliegenden Ausführungsbeispiels weist die Konsole 5 eine Eingabeeinrichtung 15 auf, mittels derer die das Lebewesen 13 behandelnde Person einen Bereich 17 oder eine Stelle in dem mittels der Anzeigevorrichtung 6 dargestellten Bildes 16 kennzeichnen kann. Die Eingabeeinrichtung 15 ist z.B. eine Rechner-Maus, insbesondere eine 3D-Maus, mittels derer ein in das Bild 16 eingeblendeter Cursor 18 bewegt werden kann. Somit kann der Cursor 18 mittels der Eingabeeinrichtung 15 an denjenigen Bereich 17 herangeführt werden, um diesen Bereich 17 z.B. durch Anklicken zu kennzeichnen. Es ist auch möglich, dass für das Kennzeichnen des Bereichs 17 wenigstens eines der Eingabevorrichtungen 7, 8 verwendet werden kann. In diesem Fall kann es vorgesehen sein, die Funktionalität der relevanten Eingabevorrichtung 7, 8 umschaltbar auszuführen, sodass mit der entsprechenden Eingabevorrichtung 7, 8 entweder der Cursor 18 bewegt werden kann oder der relevante Roboterarm 2, 3 telemanipuliert werden kann.

Im Falle des vorliegenden Ausführungsbeispiels kann der medizinische Arbeitsplatz 1 in einem zweiten Betriebsmodus betrieben werden. Für den zweiten Betriebsmodus ist z.B. die Steuervorrichtung 4 derart eingerichtet, bzw. läuft auf dieser ein geeignetes Rechenprogramm, sodass aufgrund des gekennzeichneten Bereichs 17 die Steuervorrichtung 4 automatisch die Antriebe des Roboterarms 3 ansteuert, an dem das medizinische Instrument 11 befestigt ist, sodass das medizinische Instrument 11 automatisch den Bereich des Lebewesens 13 behandelt, der dem im Bild 16 gekennzeichneten Bereich 17 entspricht. Dazu läuft z.B. auf der Steuervorrichtung 4 ein geeignetes Bildverarbeitungsprogramm, das das Bild 16 bzw. den im Bild 16 gekennzeichneten Bereich 17 analysiert und anschließend die Antriebe des Roboterarms 3 derart ansteuert, sodass die Befestigungsvorrichtung 10, an dem das medizinische Instrument 11 befestigt ist, derart bewegt wird, dass das medizinische Instrument 11 an den Bereich des Lebewesens 13 heran bewegt wird, der dem gekennzeichneten Bereich 17 entspricht. Anschließend steuert die Steuervorrichtung 4 das medizinische Instrument 11 derart an, dass dieses eine entsprechende Behandlung am Lebewesen 13 durchführt. Die Berechnung der Bewegung für den relevanten Roboterarm 3 kann auch zusätzlich basierend auf den präoperativen Daten und/oder den Daten der anatomischen Atlanten erfolgen. Die präoperativen Daten können z.B. auch segmentiert sein oder werden.

Es kann möglich sein, den medizinischen Arbeitsplatz 1 mit verschiedenen medizinischen Instrumenten zu versehen, die mit einem der Roboterarme 2, 3 im ersten Betriebsmodus telemanipuliert bewegt werden. Für den zweiten Betriebsmodus kann es vorgesehen sein, dass die Art des verwendeten medizinischen Instruments für die automatische Behandlung des gekennzeichneten Bereichs 17 manuell wählbar ist. Dazu kann z.B. in das Bild 16 eine Auswahlliste 19 eingeblendet werden, die eine Mehrzahl verschiedener medizinischer Instrumente zur Auswahl anbietet, von denen eines mittels des Cursors 18 angeklickt werden kann, um ausgewählt zu werden. Die Auswahl des medizintechnischen Instruments kann auch mittels Gestenerkennung, Spracheingabe, etc. erfolgen.

## Patentansprüche

1. Medizinischer Arbeitsplatz zum Behandeln eines Lebewesens (13) mittels eines medizinischen Instruments (11), aufweisend
- wenigstens einen Roboterarm (3), der mehrere mittels Gelenken verbundene Glieder, Antriebe zum Bewegen der Glieder, und eine Befestigungsvorrichtung (10) aufweist, die vorgesehen ist, mit dem medizinischen Instrument (11) zum Behandeln des Lebewesens (13) versehen zu werden,
- wenigstens eine mit den Antrieben gekoppelte Steuervorrichtung (4), welche eingerichtet ist, Signale zum Ansteuern der Antriebe zu erzeugen, damit die Befestigungsvorrichtung (10) den Signalen zugeordnete Bewegungen durchführt,
- eine mit der Steuervorrichtung (4) gekoppelte Anzeigevorrichtung (6) zum Anzeigen wenigstens eines während der Behandlung des Lebewesens (13) aufgenommenen Bildes (16) vom Behandlungsbereich des Lebewesens (13) aufweist,
- eine Eingabeeinrichtung (15), mittels derer ein Bereich (17) im Bild (16) kennzeichenbar ist, wobei die Steuervorrichtung (4) eingerichtet ist, in einem Betriebsmodus aufgrund des gekennzeichneten Bereichs (17) automatisch eine Bewegung des Roboterarms (3) zu errechnen und die Antriebe dieses Roboterarms (3) entsprechend anzusteuern, sodass das medizinische Instrument (11) mittels dieses Roboterarms (3) automatisch den Bereich des Lebewesens (13) behandelt, der dem gekennzeichneten Bereich (17) zugeordnet ist, und
- eine mit der Steuervorrichtung (4) gekoppelte Bedienkonsole (5), die eine mit der Steuervorrichtung (4) gekoppelte manuelle Eingabevorrichtung (7, 8) aufweist, wobei die Steuervorrichtung (4) in einem weiteren Betriebsmodus die Signale aufgrund eines manuellen Bewegens der Eingabevorrichtung (7, 8) erzeugt, sodass der Roboterarm (3) eine der manuellen Bewegung entsprechende telemanipulierte Bewegung durchführt,
**dadurch gekennzeichnet, dass** bei dem medizinischen Arbeitsplatz wenigstens eine der manuellen Eingabevorrichtungen (7, 8) umschaltbar ausgeführt ist, sodass diese Eingabevorrichtung (7, 8) im weiteren Betriebsmodus zum telemanipulierten Bewegen und im Betriebsmodus zum Kennzeichnen des Bereichs (17) verwendbar ist.

2. Medizinischer Arbeitsplatz nach Anspruch 1, dessen Bedienkonsole (5) die Anzeigevorrichtung (6) aufweist.

3. Medizinischer Arbeitsplatz nach Anspruch 1 oder 2, aufweisend eine Kamera (21), insbesondere eine mittels eines Endoskops (20) geführte Kamera (21), die vorgesehen ist, an eine Befestigungsvorrichtung (9) eines weiteren Roboterarms (2) des medizinischen Arbeitsplatzes (1) befestigt zu werden, um das Bild (16) vom Behandlungsbereich des Lebewesens (13) während der Behandlung zu erstellen.

4. Medizinischer Arbeitsplatz nach einem der Ansprüche 1 bis 3, bei dem das Bild (16) ein dreidimensionales Bild ist und/oder die Anzeigevorrichtung (6) eingerichtet ist, ein dreidimensionales Bild darzustellen.

5. Medizinischer Arbeitsplatz nach einem der Ansprüche 1 bis 4, bei dem die Eingabeeinrichtung (15) als eine Rechner-Maus, insbesondere als eine 3D-Maus ausgebildet ist.

6. Medizinischer Arbeitsplatz nach einem der Ansprüche 1 bis 5, aufweisend Eingabemittel, die vorgesehen sind, die Art des medizinischen Instruments (11) auszuwählen.

7. Medizinischer Arbeitsplatz nach Anspruch 6, bei dem die Eingabemittel auf Spracheingabe und/oder auf Gestenerkennung basieren.

8. Medizinischer Arbeitsplatz nach einem der Ansprüche 1 bis 7, bei dem das medizinische Instrument (11) zum Kleben, Nähen, Klammern, Schneiden oder Cautern, oder für eine Biopsie oder für die Brachytherapie vorgesehen ist.

9. Medizinischer Arbeitsplatz nach einem der Ansprüche 1 bis 8, dessen Steuervorrichtung (4) eingerichtet ist, die Bewegung des Roboterarms (3) aufgrund von vor der Behandlung aufgenommener Bilder des Lebewesens (13) und/oder aufgrund von anatomischen Atlanten zu errechnen.

## Claims

1. Medical workstation for treating a living being (13) by means of a medical instrument (11), comprising:
- at least one robot arm (3), which comprises a plurality of links connected by joints, drives for moving the links and a securing device (10) which is provided with the medical instrument (11) for treating the living being (13),
- at least one control device (4) coupled to the drives, which is configured to generate signals for controlling the drives, so that the securing device (10) executes movements assigned to the signals,
- a display device (6) coupled to the control device (4) for displaying at least one image (16) of the treatment area of the living being (13) taken during the treatment of the living being (13),
- an input device (15), by means of which an area (17) in the image (16) can be marked, wherein the control device (4) is configured in an operating mode due to the marked area (17) to automatically calculate a movement of the robot arm (3) and to control the drives of said robot arm (3) accordingly so that by means of this robot arm (3) the medical instrument (11) automatically treats the area of the living being (13) which is assigned to the marked area (17), and
- an operating console (5) coupled to the control device (4), which operating console has a manual input device (7, 8) coupled to the control device (4), wherein the control device (4) in a further operating mode generates the signals on the basis of a manual movement of the input device (7, 8) so that the robot arm (3) performs a telemanipulated movement corresponding to the manual movement, **characterised in that** in the medical workstation at least one of the manual input devices (7, 8) is designed to be switchable, so that said input device (7, 8) can be used in the further operating mode for telemanipulated movement and in the operating mode for marking the area (17).

2. Medical workstation according to claim 1, the operating console (5) of which comprises the display device (6).

3. Medical workstation according to claim 1 or 2, comprising a camera (21), in particular a camera (21) guided by means of an endoscope (20) which camera is provided to be secured onto a securing device (9) of a further robot arm (2) of the medical workstation (1) in order to create the image (16) of the treatment area of the living being (13) during the treatment.

4. Medical workstation according to any of claims 1 to 3, wherein the image (16) is a three-dimensional image and/or the display device (6) is configured to represent a three-dimensional image.

5. Medical workstation according to any of claims 1 to 4, wherein the input device (15) is designed as a computer mouse, in particular as a 3D mouse.

6. Medical workstation according to any of claims 1 to 5, comprising input means which are provided to select the type of medical instrument (11).

7. Medical workstation according to claim 6, wherein the input means are based on speech input and/or on gesture recognition.

8. Medical workstation according to any of claims 1 to 7, wherein the medical instrument (11) is provided for sticking, stitching, clamping, cutting or cauterising or for a biopsy or for the brachytherapy.

9. Medical workstation according to any of claims 1 to 8, the control device (4) of which is configured to calculate the movement of the robot arm (3) based on images of the living being (13) taken prior to the treatment and/or based on anatomical atlases.

## Revendications

1. Poste de travail médical pour le traitement d'un être vivant (13) au moyen d'un instrument médical (11), comprenant
- au moins un bras de robot (3) comprenant une pluralité de membres reliés au moyen d'articulations, des entraînements pour déplacer les membres, et un dispositif de fixation (10) qui est prévu pour être muni de l'instrument médical (11) pour le traitement de l'être vivant (13),
- au moins un dispositif de commande (4) couplé aux entraînements, qui est configuré pour générer des signaux pour piloter les entraînements de telle sorte que le dispositif de fixation (10) effectue des mouvements associés aux signaux,
- un dispositif d'affichage (6) couplé au dispositif de commande (4) pour afficher au moins une image (16) de la zone de traitement de l'être vivant (13), prise pendant le traitement de l'être vivant (13),
- un dispositif de saisie (15) au moyen duquel une zone (17) dans l'image (16) peut être marquée, le dispositif de commande (4) étant configuré pour calculer automatiquement un mouvement du bras de robot (3) dans un mode de fonctionnement sur la base de la zone marquée (17) et pour piloter de manière correspondante les entraînements de ce bras de robot (3), de telle sorte que l'instrument médical (11) traite automatiquement la zone de l'être vivant (13) qui est associée à la zone marquée (17), et
- un pupitre de commande (5) couplé au dispositif de commande (4), qui présente un dispositif de saisie manuelle (7, 8) couplé au dispositif de commande (4), le dispositif de commande (4) générant, dans un autre mode de fonctionnement, les signaux sur la base d'un mouvement manuel du dispositif de saisie (7, 8), de telle sorte que le bras de robot (3) effectue un mouvement télémanipulé correspondant au mouvement manuel, **caractérisé en ce que** sur le poste de travail médical, au moins l'un des dispositifs de saisie manuelle (7, 8) est réalisé de manière à pouvoir être commuté, de telle sorte que ce dispositif de saisie (7, 8) peut être utilisé dans l'autre mode de fonctionnement pour effectuer le mouvement télémanipulé et dans le mode de fonctionnement pour le marquage de la zone (17).

2. Poste de travail médical selon la revendication 1, dont le pupitre de commande (5) présente le dispositif d'affichage (6).

3. Poste de travail médical selon la revendication 1 ou 2, présentant une caméra (21), en particulier une caméra (21) guidée au moyen d'un endoscope (20), qui est prévue pour être fixée à un dispositif de fixation (9) d'un autre bras de robot (2) du poste de travail médical (1) afin de produire l'image (16) de la zone de traitement de l'être vivant (13) pendant le traitement.

4. Poste de travail médical selon l'une des revendications 1 à 3, dans lequel l'image (16) est une image tridimensionnelle et/ou le dispositif d'affichage (6) est configuré pour afficher une image tridimensionnelle.

5. Poste de travail médical selon l'une des revendications 1 à 4, dans lequel le dispositif de saisie (15) est réalisé sous la forme d'une souris d'ordinateur, en particulier d'une souris 3D.

6. Poste de travail médical selon l'une des revendications 1 à 5, présentant des moyens de saisie qui sont prévus pour sélectionner le type d'instrument médical (11).

7. Poste de travail médical selon la revendication 6, dans lequel les moyens de saisie sont basés sur la saisie de la parole et/ou la reconnaissance de gestes.

8. Poste de travail médical selon l'une des revendications 1 à 7, dans lequel l'instrument médical (11) est prévu pour coller, suturer, agrafer, couper ou cautériser, ou pour une biopsie ou pour une curiethérapie.

9. Poste de travail médical selon l'une des revendications 1 à 8, dont le dispositif de commande (4) est configuré pour calculer le mouvement du bras de robot (3) sur la base d'images de l'être vivant (13) prises avant le traitement et/ou sur la base d'atlas anatomiques.
